# EUROPEAN PATENT APPLICATION

(11) **EP 2 735 560 A1**
(43) Date of publication of application: **28.05.2014**
(21) Application number: 12814649.5
(22) Date of filing: 18.07.2012
(51) Int. Cl.: C07D 207/16

(54) **METHOD FOR PRODUCING OPTICALLY ACTIVE 2-METHYLPROLINE DERIVATIVE**

(30) Priority: 20.07.2011 JP 2011159364
(71) Applicant: Kaneka Corporation, Osaka-shi, Osaka 530-8288 (JP)
(72) Inventor: NISHIYAMA, Akira, Takasago-shi Hyogo 676-8688 (JP)
(74) Representative: Vossius & Partner
(86) International application number: PCT/JP2012/068187
(87) International publication number: WO 2013/011999

(57) **Abstract**

An optically active 2-methylproline derivative is produced by forming a salt from a racemic 2-methylproline derivative and an optically active 1-arylethylamine derivative and precipitating the salt in a form of solid from a solvent.

## Description

### TECHNICAL FIELD

The present invention relates to a method for producing an optically active 2-methylproline derivative which is useful as a pharmaceutical intermediate.

### BACKGROUND ART

As a method for producing an optically active 2-methylproline derivative, the following methods are known:
Prior art 1: a method for obtaining N-(benzyloxycarbonyl)-2-methyl-L-proline (Non-patent Document 1); and
Prior art 2: a method for producing N-(tert-butoxycarbonyl)-2-methyl-D-proline (Patent Document 1).

In Prior art 1, a salt is formed from racemic N-(benzyloxycarbonyl)-2-methylproline and kinin, and the salt is precipitated from acetone to obtain a kinin salt of N-(benzyloxycarbonyl)-2-methyl-L-proline. Then, the kinin salt is purified by repeating recrystallization 6 times using a mixed solvent of acetone / diethyl ether, and the purified kinin salt is dissociated using ammonia water to obtain N-(benzyloxycarbonyl)-2-methyl-L-proline.

In Prior art 2, L-alanine benzyl ester is reacted with 1-bromo-3-chloropropane and further protected by Boc group to obtain N-(3-chloropropyl)-N-(tert-butoxycarbonyl)-L-alanine benzyl ester. Then, the obtained ester is cyclized by treating with lithium hexamethyldisilazide in dimethylformamide and subjecting to hydrogenolysis in the presence of a palladium catalyst, to produce N-(tert-butoxycarbonyl)-2-methyl-D-proline.

### PRIOR ART

### PATENT DOCUMENT

Patent Document 1: JP 2008-535926 T

### NON-PATENT DOCUMENT

Non-patent Document 1: Journal of Polymer Science, 1977, 15, 1413-1421

### DISCLOSURE OF THE INVENTION

### PROBLEMS TO BE SOLVED BY THE INVENTION

The method of Prior Art 1 requires many times, actually 6 times, of recrystallization processes for resolution. Therefore, the method is not an effective resolution method. As such an example, a method is not known in which a reagent for optical resolution is used and by which an optically active 2-methylproline derivative having high optical purity can be obtained without difficulty.

In addition, the method of Prior Art 2 is not necessarily advantageous to industrial production of an optically active 2-methylproline derivative, since expensive base and palladium catalyst are used for the production.

### MEANS FOR SOLVING THE PROBLEMS

The inventor of the present invention intensively studied. As a result, the inventor completed the present invention by finding out the method for producing an optically active 2-methylproline derivative without difficulty.

The present invention relates to a method for producing a compound represented by the following formula (3): 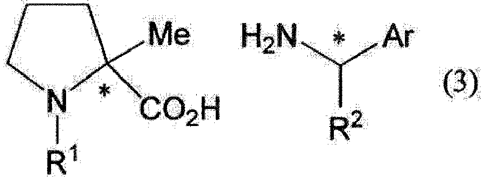 wherein R¹ is a hydrogen atom or a protective group for the amino group; R² is a C₁₋₁₂ alkyl group or a C₇₋₁₂ aralkyl group; Ar is a C₆₋₁₂ aryl group; and * indicates an asymmetric carbon atom,
comprising the steps of
forming a salt from a racemic 2-methylproline derivative represented by the following formula (1): 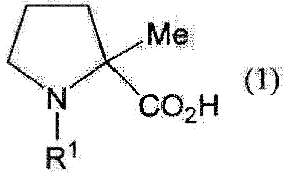 wherein R¹ is the same as the above,
and an optically active 1-arylethylamine derivative represented by the following formula (2): 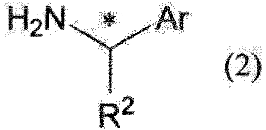 wherein R², Ar and * are the same as the above,
and precipitating the compound (3) as a salt in a form of solid from a solvent.

Also, the present invention relates to an optically active 2-methylproline derivative represented by the following formula (3) : 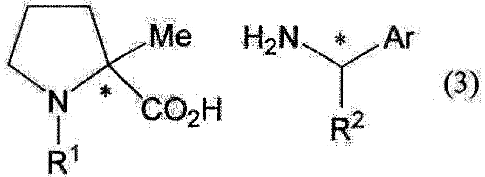 wherein R¹ is a hydrogen atom or a protective group for the amino group; R² is a C₁₋₁₂ alkyl group or a C₇₋₁₂ aralkyl group; Ar is a C₆₋₁₂ aryl group; and * indicates an asymmetric carbon atom.

### EFFECT OF THE INVENTION

According to the method of the present invention, an optically active 2-methylproline derivative can be easily produced.

### MODE FOR CARRYING OUT THE INVENTION

Hereinafter, the present invention is described in detail.

A racemic 2-methylproline derivative which is a starting material compound of the present invention is represented by the following formula (1) :

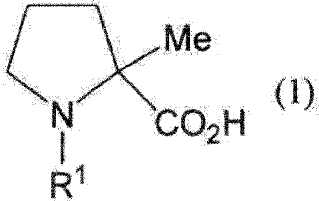

In the above formula, R¹ is a hydrogen atom or a protective group for the amino group. Specifically, the protective group for the amino group is exemplified by protective groups described in Theodora W. Greene, "Protective Groups in Organic Synthesis, Third edition" published by John Wiley & Sons, pp.494-653. The protective group for the amino group is preferably a carbamate type protective group such as methoxycarbonyl group, ethoxycarbonyl group, isopropyloxycarbonyl group, tert-butoxycarbonyl group, phenyloxycarbonyl group, benzyloxycarbonyl group and p-nitrobenzyloxycarbonyl group; and an acyl type protective group such as acetyl group, trifluoroacetyl group, pivaloyl group and benzoyl group. The protective group for the amino group is more preferably a carbamate type protective group such as methoxycarbonyl group, ethoxycarbonyl group, isopropyloxycarbonyl group, tert-butoxycarbonyl group, phenyloxycarbonyl group, benzyloxycarbonyl group and p-nitrobenzyloxycarbonyl group, and particularly preferably tert-butoxycarbonyl group.

For example, the above-described compound (1) can be produced by the method described in J. Med. Chem., 2009, 52, 514-523. Specifically, N-(benzyloxycarbonyl)-proline methyl ester isα-methylated by treating with sodium bis(hexamethyl)disilazane and methyl iodide, and then the corresponding N-(benzyloxycarbonyl)-2-methylproline is produced by alkaline hydrolysis.

The optically active 1-arylethylamine derivative used in the present invention is represented by the following formula (2):

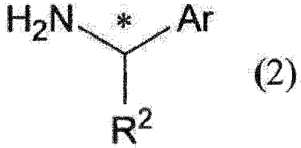

In the above formula, R² is a C₁₋₁₂ alkyl group or a C₇₋₁₂ aralkyl group. The groups may be substituted by 1 or more substituents. Such a substituent is exemplified by a halogen atom such as fluorine atom, chlorine atom, bromine atom and iodine atom; an alkoxy group such as methoxy group and ethoxy group; methylthio group; trifluoromethyl group; acetyl group; benzoyl group; cyano group; nitro group; carboxy group; alkoxycarbonyl group such as methoxycarbonyl group and ethoxycarbonyl group.

Specifically, R² is exemplified by methyl group, ethyl group, n-propyl group, isopropyl group, cyclopropyl group, n-butyl group, isobutyl group, sec-butyl group, tert-butyl group, cyclobutyl group, n-pentyl group, cyclohexyl group and benzyl group, and is preferably methyl group or ethyl group, and more preferably methyl group.

Ar is a C₆₋₁₂ aryl group and may be substituted by 1 or more substituents. Such a substituent is exemplified by the same substituents for R².

Ar is specifically exemplified by phenyl group, p-methylphenyl group, p-chlorophenyl group, p-methoxyphenyl group, p-nitrophenyl group, 3,5-bis(trifluoromethyl)phenyl group, 1-naphthyl group and 2-naphthyl group, preferably phenyl group or p-chlorophenyl group, and more preferably phenyl group.

The symbol "*" indicates an asymmetric carbon atom.

The above-described optically active 1-arylethylamine derivative is preferably (S)-1-phenethylamine, (S)-1-(p-chlorophenyl)ethylamine, (S)-1-phenylpropylamine, (R)-1-phenethylamine, (R)-1-(p-chlorophenyl)ethylamine, (R)-1-phenylpropylamine.

The optical purity of the above-described optically active 1-arylethylamine derivative is, for example, not less than 90%ee, preferably not less than 95%ee, more preferably not less than 98%ee, and particularly preferably 100%ee.

The optically active 1-arylethylamine derivative may be produced by a publicly known method. Alternatively, a commercially available optically active 1-arylethylamine derivative may be directly used. Further, such optically active 1-arylethylamine derivatives may be properly derivatized according to a publicly known organic synthesis method. In the present invention, after the optically active 2-methylproline derivative represented by the following formula (3) is obtained, the compound may be salt-dissociated to obtain a salt-dissociated compound, i.e. an optically active 2-methylproline derivative. During such a salt-dissociation, the optically active 1-arylethylamine derivative is regenerated. The regenerated derivative may be used as a raw material compound again in order to obtain the salt. When the optically active 1-arylethylamine derivative is reused, the optically active 2-methylproline derivative salt may be produced with low cost even if production cost of the optically active 1-arylethylamine derivative is high.

The optically active 2-methylproline derivative obtained by the present invention is represented by the following formula (3): 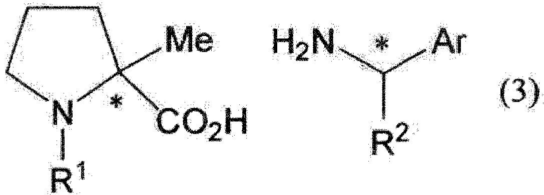 Wherein R¹, R², Ar and * are the same as the above.

The above-described compound (3) is preferably N-(tert-butoxycarbonyl)-2-methyl-L-proline / (S)-1-phenethylamine salt, N-(tert-butoxycarbonyl)-2-methyl-D-proline / (R)-1-phenethylamine salt, N-(tert-butoxycarbonyl)-2-methyl-L-proline / (S)-1-(p-chlorophenyl)ethylamine salt, N-(tert-butoxycarbonyl)-2-methyl-D-proline / (R)-1-(p-chlorophenyl)ethylamine salt, N-(tert-butoxycarbonyl)-2-methyl-L-proline / (S)-1-phenylpropylamine salt, N-(tert-butoxycarbonyl)-2-methyl-D-proline / (R)-1-phenylpropylamine salt, and more preferably N-(tert-butoxycarbonyl)-2-methyl-L-proline / (S)-1-phenethylamine salt, N-(tert-butoxycarbonyl)-2-methyl-D-proline / (R)-1-phenethylamine salt.

The above-described compound (3) is a novel compound and has not been described in any documents.

Then, a method is described in which a salt is formed from the racemic 2-methylproline derivative represented by the above formula (1) and the optically active 1-arylethylamine derivative represented by the above formula (2), and the optically active 2-methylproline derivative represented by the above formula (3) is precipitated in a form of solid from a solvent.

An amount of the above-described compound (2) to be used relative to 1 mole of the above-described compound (1) is preferably not less than 0.1 times by mole and not more than 2 times by mole, and more preferably not less than 0.5 times by mole and not more than 1 time by mole. An amount of the compound (2) to be used may be larger or smaller than an amount of the compound (1) to be used or similar to an amount of the compound (1) to be used. When the amount of the compound (2) is larger, the amount relative to 1 mole of the compound (1) is exemplified by not less than 1.01 times by mole and not more than 2 times by mole, and preferably not less than 1.05 times by mole and not more than 1.5 times by mole. When the amount of the compound (2) is similar, the amount relative to 1 mole of the compound (1) is exemplified by not less than 0.8 times by mole and not more than 1.2 times by mole, and preferably not less than 0.9 times by mole and not more than 1.1 times by mole. When the amount of the compound (2) is smaller, the amount relative to 1 mole of the compound (1) is exemplified by not less than 0.5 times by mole and not more than 0.95 times by mole, and preferably not less than 0.7 times by mole and not more than 0. 9 times by mole. When the compound (2) is used in an excessive amount, the compound (1) can be certainly converted to the salt and efficiency can be improved. In addition, even when the compound (2) is used in an excessive amount, a loss is not caused since the compound (2) can be recovered in the subsequent steps.

In order to precipitate the solid, for example, the compound (1) and the compound (2) are mixed in a solvent as the solid precipitation methods (a) to (e) described later.

A solvent to be used is not particularly limited, and specifically exemplified by water; an alcohol solvent such as methanol, ethanol, n-propanol, isopropanol, n-butanol, tert-butanol and ethylene glycol; an ester solvent such as ethyl acetate, n-propyl acetate and isopropyl acetate; an ether solvent such as tetrahydrofuran, diethyl ether, 1,4-dioxane, methyl tert-butyl ether, diisopropyl ether and ethylene glycol dimethyl ether; a ketone solvent such as acetone and methyl ethyl ketone; a nitrile solvent such as acetonitrile and propionitrile; an aromatic hydrocarbon solvent such as benzene, toluene and xylene; an aliphatic hydrocarbon solvent such as pentane, hexane, heptane, cyclohexane and methylcyclohexane; a halogen solvent such as methylene chloride, 1,2-dichloroethane and chlorobenzene; a sulfoxide solvent such as dimethylsulfoxide; an amide solvent such as N,N-dimethylformamide and N,N-dimethylacetamide; a urea solvent such as dimethylpropyleneurea; a phosphorous triamide solvent such as hexamethylphosphorous triamide. One of the above solvents may be used alone; alternatively, two or more solvents may be used in combination. When two or more solvents are used in combination, a mixing ratio thereof is not particularly limited.

The solvent is preferably isopropanol, ethyl acetate, isopropyl acetate, tetrahydrofuran, methyl tert-butyl ether, diethyl ether, acetone, methyl ethyl ketone, acetonitrile, toluene, methylene chloride, chlorobenzene, hexane, heptane, cyclohexane or methylcyclohexane, more preferably isopropanol, ethyl acetate, tetrahydrofuran, acetone, acetonitrile, toluene, methylene chloride, and particularly preferably acetone.

An amount of the solvent to be used relative to 1 part by weight of the compound (1) is preferably not more than 50 parts by weight, and more preferably not more than 20 parts by weight, since excessive amount of the solvent is not preferred in terms of cost and after-treatment. The lower limit of a use amount of the solvent is not particularly limited as long as the precipitation of the solid is not inhibited, and for example, the amount relative to 1 part by weight of the compound (1) may be not less than 1 part by weight or not less than 5 parts by weight, and particularly not less than 10 times by weight.

In the present invention, a method for precipitating the compound (3) in a form of solid is not particularly limited, and exemplified by the following methods:
(a) the compound (1) and the compound (2) are mixed in the solvent to precipitate the solid;
(b) the compound (1) and the compound (2) are mixed in the solvent, and then the mixture is cooled to precipitate the solid;
(c) the compound (1) and the compound (2) are mixed in the solvent, and then the mixture is concentrated to precipitate the solid;
(d) the compound (1) and the compound (2) are mixed in the solvent, and then a poor solvent is further added thereto to precipitate the solid;
(e) the compound (1) and the compound (2) are mixed in the solvent, and then the solvent is distilled off and replaced by a poor solvent to precipitate the solid.

In order to precipitate the solid, the above methods (a) to (e) may be properly combined. In addition, a seed solid may be added when the solid is precipitated.

As the poor solvent which is used in the methods (d) and (e), an aliphatic hydrocarbon solvent such as hexane and heptane is exemplified.

A temperature in the methods (a) to (e) to precipitate the solid is not particularly limited and may be properly determined depending on the kind of the precipitated solid and the kind of the solvent to be used. It is preferred that the temperature is set to lower than the temperature at which the compound (3) is dissolved in the used solvent or mixed solvents depending on the target precipitate amount and quality of the solid.

The compound (3) which is precipitated by the methods (a) to (e) for precipitating the solid may be separated to be obtained by filtration under reduced pressure, pressure filtration, centrifugation and the like. In addition, if a mother liquid remains in the obtained solid and an enantiomeric excess (%ee) of the solid is decreased, the solid may be further washed using an organic solvent to improve the quality as necessary.

A method for drying the solid is not particularly limited, and it is preferred the solid is dried under reduced pressure or in vacuo at about 60°C or lower to inhibit thermal decomposition or melting.

If the enantiomeric excess (%ee) is not sufficiently improved, the solid may be treated with any one of the above-described methods (a) to (e) for precipitating the solid again, or the compound (3) may be washed using a solvent, or the solid may be precipitated again by a method according to any one of the above-described methods (a) to (e).

As necessary, the compound (1) which has higher enantiomeric excess (%ee) may be obtained by salt dissociation from the compound (3) which is obtained by the above method. For example, such a salt dissociation process may be carried out by adding an acid aqueous solution to the compound (3) in order to liberate the compound (1), extracting the compound (1) using an organic solvent, and removing the extraction solvent. The acid aqueous solution is exemplified by hydrochloric acid, hydrobromic acid, sulfuric acid, acetic acid and citric acid. The organic solvent is exemplified by ethyl acetate, toluene and methyl tert-butyl ether. A method for removing the extraction solvent is exemplified by heating under reduced pressure. When the compound (1) is extracted from the acid aqueous solution using an organic solvent, the compound (2) remains in the acid aqueous solution and therefore, the compound (2) can be easily recovered to be used again.

Also, the above-described salt dissociation may be carried out contacting the compound (3) with a basic aqueous solution.

A chemical purity and an optical purity of the obtained optically active compound (1) can be further improved by crystallization if necessary. A solvent for such crystallization is exemplified by the same solvents exemplified for precipitating the compound (3) in a form of solid. The crystallization solvent is preferably an ester solvent such as ethyl acetate and an aliphatic hydrocarbon solvent such as hexane.

The compound (3) obtained by the present invention has not been described in any documents and is a novel compound. It is naturally not known that the compound (3) becomes solid. In the present invention, the term "solid" means a crystal, amorphous or a mixture thereof. A mixing ratio of the mixture is not limited.

The present application claims the benefit of the priority date of Japanese patent application No. 2011-159364 filed on July 20, 2011, and all of the contents of the Japanese patent application No. 2011-159364 filed on July 20, 2011 are incorporated by reference.

### EXAMPLES

Hereinafter, the present invention is described in more detail with Examples. However, the present invention is not intended to be limited by the Examples.

Analysis method of optical purity (isocratic method) -1
Column: CHIRALCEL OJ-RH 150 × 4.6 mm, manufactured by Daicel Chemical Industries, Ltd.
Mobile phase: acetonitrile / phosphoric acid aqueous solution (pH 2.5) = 2 / 8 by volume
Flow rate: 0.7 mL/min
Detector: UV 210 nm
Column temperature: 30°C
Retention time of N-(tert-butoxycarbonyl)-2-methyl-D-proline: 7.1 minutes
Retention time of N-(tert-butoxycarbonyl)-2-methyl-L-proline: 9.8 minutes

When the compound (3) (salt) obtained in Examples is analyzed by the above analysis method, the salt is dissociated during the analysis and an optical purity of N-(tert-butoxycarbonyl)-2-methyl-proline can be measured.

### Example 1: Production of N-(tert-butoxycarbonyl)-2-methyl-D-proline / (R)-1-phenethylamine salt

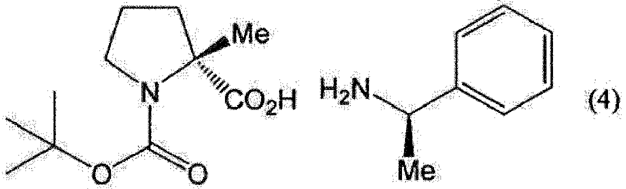

To a acetone solution (3 mL) of racemic N-(tert-butoxycarbonyl) -2-methylproline (241 mg, 94.9 wt%, 1 mmol), an acetone solution (1 mL) of (R)-1-phenethylamine (121 mg, 1 mmol) was added. After a while, a solid was precipitated. After the mixture was stirred at 25°C for 30 minutes, the solid was obtained by filtration under reduced pressure. The solid was washed using 2 mL of acetone and 4 mL of hexane, and then dried in vacuo to obtain the target compound as a white solid (amount: 131.9 mg, yield: 38%, optical purity: 97.8% ee).
¹H-NMR (CDCl₃) : δ (ppm) 1.39(s, 9H), 1.41(s, 3H), 1.50(d, 3H), 1.76-1.88(m, 3H), 2.32-2.36(m, 1H), 3.42-3.55(m, 2H), 4.22(q, 1H), 4.49(brs, 3H), 7.26-7.39(m, 5H)

### Examples 2 to 7: Production of N-(tert-butoxycarbonyl)-2-methyl-L-proline / (S)-1-phenethylamine salt

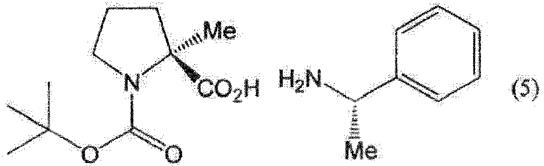

Racemic N-(tert-butoxycarbonyl)-2-methylproline (229 mg, 1 mmol) was diluted by respective solvent (4 mL) demonstrated in Table 1, and (S)-1-phenethylamine (121 mg, 1 mmol) was added thereto. After a solid was precipitated, the mixture was stirred at 25°C for 30 minutes. The solid was obtained by filtration under reduced pressure. The solid was washed using the respective solvent (2 mL), and then dried in vacuo to obtain the target compound as a white solid. The results are demonstrated in the following table.

**Table 1**

| Example | Solvent | Apparent yield | Optical purity |
|---|---|---|---|
| 2 | acetone | 43% | 92.1%ee |
| 3 | tetrahydrofuran | 44% | 94.5%ee |
| 4 | isopropanol | 29% | 96.0%ee |
| 5 | methylene chloride | 26% | 98.4%ee |
| 6 | toluene | 39% | 88.0%ee |
| 7 | ethyl acetate | 53% | 81.6%ee |

### Example 8: Production of N-(tert-butoxycarbonyl)-2-methyl-L-proline

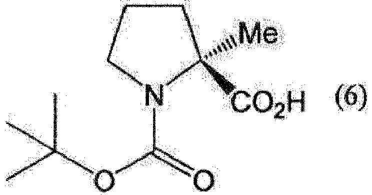

To an acetone solution (9 mL) of racemic N-(tert-butoxycarbonyl)-2-methylproline (723 mg, 94.9 wt%, 3 mmol), an acetone solution (3 mL) of (S) -1-phenethylamine (363 mg, 3 mmol) was added. After a while, a solid was precipitated. The mixture was stirred at 25°C for 3 hours, and then cooled down to 5°C. After the mixture was stirred at 5°C for 30 minutes, the solid was obtained by filtration under reduced pressure. The solid was washed using 6 mL of cooled acetone and dried in vacuo, to obtain N-(tert-butoxycarbonyl)-2-methyl-L-proline / (S)-1-phenethylamine salt as a white solid (amount: 438.1 mg, yield: 46%, optical purity: 98.7%ee).

Subsequently, ethyl acetate (6 mL) and water (2 mL) were added to the white solid, and pH was adjusted to 1 using concentrated hydrochloric acid. After the aqueous layer was separated, the organic layer was washed 2 times using 2 mL of water and concentrated under reduced pressure. To the residue, ethyl acetate (0.6 mL) and hexane (6 mL) were added to precipitate a solid. After the mixture was stirred at 5°C for 30 minutes, the solid was obtained by filtration under reduced pressure. The solid was washed using 3 mL of cooled hexane and dried in vacuo, to obtain the target compound as a white solid (yield: 78%, optical purity: 100%ee).

### Example 9: Production of N-(tert-butoxycarbonyl-)-2-methyl-D-proline / (R)-1-(p-chlorophenyl)ethylamine salt

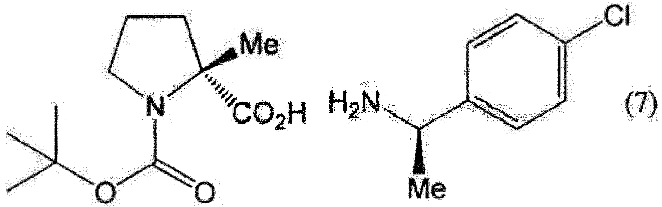

To an ethyl acetate solution (3 mL) of racemic N- (tert-butoxycarbonyl)-2-methylproline (241 mg, 94.9 wt%, 1 mmol), an ethyl acetate solution (1 mL) of (R)-1-(p-chlorophenyl)ethylamine (155.5 mg, 1 mmol) was added. After a while, a solid was precipitated. The mixture was stirred at 25°C for 1 hour, and then the solid was obtained by filtration under reduced pressure. After the solid was washed using 2 mL of ethyl acetate and 4 mL of hexane, the solid was dried in vacuo, to obtain the target compound as a white solid (amount: 114.7 mg, yield: 30%, optical purity: 100%ee).
¹H-NMR (CDCl₃) : δ (ppm) 1.37(s, 9H), 1.40(s, 3H), 1.47(d, 3H), 1.76-1.87(m, 3H), 2.25-2.29(m, 1H), 3.43-3.54(m, 2H), 4.21(q, 1H), 5.01(brs, 3H), 7.27(d, 2H), 7.35(d, 2H)

### Example 10: Production of N-(tert-butoxycarbonyl)-2-methyl-L-proline / (S)-1-phenylpropylamine salt

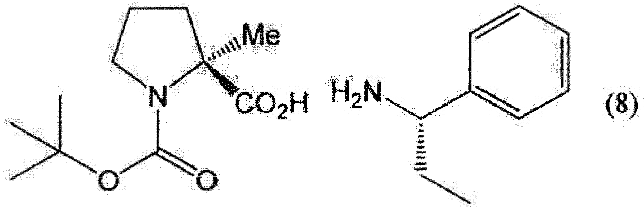

To an ethyl acetate solution (3 mL) of racemic N- (tert-butoxycarbonyl)-2-methylproline (241 mg, 94.9 wt%, 1 mmol), an ethyl acetate solution (1 mL) of (S)-1-phenylpropylamine (135 mg, 1 mmol) was added. After a while, a solid was precipitated. The mixture was stirred at 25°C for 1 hour, and then the solid was obtained by filtration under reduced pressure. After the solid was washed using 2 mL of ethyl acetate and 4 mL of hexane, the solid was dried in vacuo, to obtain the target compound as a white solid (amount: 159.3 mg, yield: 44%, optical purity: 98.3%ee). ¹H-NMR(CDCl₃) : δ (ppm) 0.86(t, 3H), 1.39(s, 9H), 1.42(s, 3H), 1.76-1.91(m, 5H), 2.29(m, 1H), 3.43-3.50(m, 2H), 3.91(q, 1H), 5.45(brs, 3H), 7.25-7.37(m, 5H)

### INDUSTRIAL APPLICABILITY

The optically active 2-methylproline derivative produced by the present invention is useful as a pharmaceutical intermediate.

## Claims

1. A method for producing a compound represented by the following formula (3): 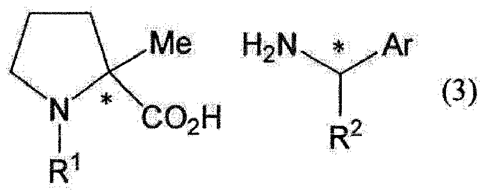 wherein R¹ is a hydrogen atom or a protective group for the amino group; R² is a C₁₋₁₂ alkyl group or a C₇₋₁₂ aralkyl group; Ar is a C₆₋₁₂ aryl group; and * indicates an asymmetric carbon atom,
comprising the steps of
forming a salt from a racemic 2-methylproline derivative represented by the following formula (1): 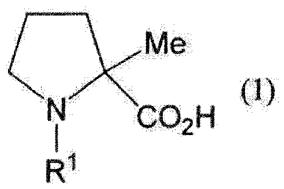 wherein R¹ is the same as the above,
and an optically active 1-arylethylamine derivative represented by the following formula (2): 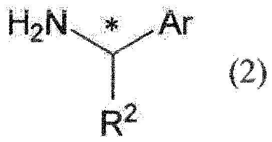 wherein R², Ar and * are the same as the above,
and precipitating the compound (3) as a salt in a form of solid from a solvent.

2. The method according to claim 1, wherein R¹ is a tert-butoxycarbonyl group.

3. The method according to claim 1 or 2, wherein R² is a methyl group or an ethyl group, and Ar is a phenyl group or a p-chlorophenyl group.

4. A method for producing the compound (1) having higher optical purity, comprising the step of subjecting the compound (3) produced by the method according to any one of claims 1 to 3 to salt dissociation process.

5. An optically active 2-methylproline derivative represented by the following formula (3): 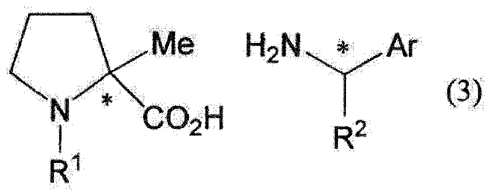 wherein R¹ is a hydrogen atom or a protective group for the amino group; R² is a C₁₋₁₂ alkyl group or a C₇₋₁₂ aralkyl group; Ar is a C₆₋₁₂ aryl group; and * indicates an asymmetric carbon atom.

6. The optically active 2-methylproline derivative according to claim 5, wherein R¹ is a tert-butoxycarbonyl group, R² is a methyl group or an ethyl group, and Ar is a phenyl group or a p-chlorophenyl group.

7. The optically active 2-methylproline derivative according to claim 5, wherein the compound (3) is N-(tert-butoxycarbonyl)-2-methyl-L-proline / (S)-1-phenethylamine salt, N-(tert-butoxycarbonyl)-2-methyl-D-proline / (R)-1-phenethylamine salt,
N-(tert-butoxycarbonyl)-2-methyl-L-proline / (S)-1-(p-chlorophenyl)ethylamine salt, N-(tert-butoxycarbonyl)-2-methyl-D-proline / (R)-1-(p-chlorophenyl)ethylamine salt, N-(tert-butoxycarbonyl)-2-methyl-L-proline / (S)-1-phenylpropylamine salt or N-(tert-butoxycarbonyl)-2-methyl-D-proline / (R)-1-phenylpropylamine salt.
